# EUROPEAN PATENT SPECIFICATION

(11) **EP 0 879 301 B1**
(45) Date of publication and mention of the grant of the patent: **07.05.2003**
(21) Application number: 97904756.0
(22) Date of filing: 30.01.1997
(51) Int. Cl.: C22B 15/00, C07C 49/12, C22B 3/26, C22B 3/30, C07C 49/76

(54) **IMPROVED BETA-DIKETONES FOR THE EXTRACTION OF COPPER FROM AQUEOUS AMMONIACAL SOLUTIONS**
BETA-DIKETONE FÜR DIE ABSCHEIDUNG VON KUPFER AUS WÄSSRIGEN AMMONIUMLÖSUNGEN
BETA-DICETONES AMELIOREES POUR L'EXTRACTION DE CUIVRE CONTENU DANS DES SOLUTIONS AQUEUSES AMMONIACALES

(30) Priority: 06.02.1996 US 11207 P; 08.01.1997 US 780759
(43) Date of publication of application: 25.11.1998
(60) Divisional application: 01116257.5
(73) Proprietor: Cognis Corporation, Gulph Mills, PA 19406 (US)
(72) Inventor: VIRNIG, Michael, J., Tucson, AZ 85745 (US); KORDOSKY, Gary, A., Tucson, AZ 85745 (US)
(74) Representative: von Kreisler, Alek, Dipl.-Chem.
(86) International application number: US9700541
(87) International publication number: WO97029215

(56) References cited:
- EP-A- 0 036 401
- WO-A-93/04208
- FR-A- 2 355 064
- US-A- 3 832 162
- US-A- 3 988 151
- US-A- 4 065 502
- US-A- 4 175 012
- US-A- 4 205 048
- US-A- 4 350 667
- US-A- 4 544 532
- US-A- 5 015 777
- J. INORG. NUCL. CHEM., 1976, Vol. 38, KOSHIMURA H., "Effect of Substituents on the Solubilities of Alkyl-Substituted Beta-Diketone Copper Chelates", pages 1705-12.
- SEP. SCI. TECHNOL., 1992, 27(12), MICKLER W. et al., "Liquid-Liquid Extraction of Copper From Ammoniacal Solution with Beta-Diketones", pages 1669-74.

## Description

### Field of the invention

This invention relates to beta-diketones and a process for the extraction of copper from aqueous ammoniacal solutions containing copper values, resulting from commercial processes, including, but not limited to, leaching of copper containing ores, such as sulfidic ores, or concentrates resulting from flotation of such sulfidic ores.

### Statement of Related Art

Practice in the recovery of copper from its sulfidic ores involves subjecting the ores to a froth flotation operation to produce a concentrate of the valuable metal sulfides and to reject the flotation tailings of valueless sulfides, silicates, aluminates and the like. One of such concentrates provided is a chalcocite concentrate containing chalcocite and covellite.

In US -A- 4,022,866 to Kuhn and Arbiter, and in their subsequent paper, "Physical and Chemical Separations via the Arbiter process" 11th International Mining Congress, April, 1975, Cagliari, Italy; proc.-Int. Miner. Process. Congress., paper 30; pp.831-847; there is described the leaching of copper sulfide concentrates with ammonia/ammonium sulfate and oxygen whereby the sulfate, and the dissolved copper may then be recovered by solvent extraction. The solvent extraction reagents are described in the patent only generally as those which preferentially load copper from alkaline solutions. In the paper, which describes the Arbiter process, the focus is on complete or nearly complete leaching of the concentrate. Another Kuhn and Arbiter paper, "Anaconda's Arbiter Process for Copper", Hydrometallurgy, CIM Bulletin, Feb, 1974, contains similar description.

U.S. -A- 4,563,256 describes a solvent extraction process for the recovery of zinc values from ammoniacal solutions, which may also contain copper values, using or employing various oxime extractants.

U.S. -A- 2,727.818 describes a method of leaching copper sulfide materials with ammoniacal leach solutions. No solvent extraction is discussed.

U.S. -A- 4,065,502 and 4,175,012 describes beta-diketones which may be employed as metal extractants in a liquid-liquid ion exchange process for recovery of metals, such as nickel or copper, from aqueous solutions containing the metal values, including aqueous ammoniacal solutions.

U.S. -A- 4,350,661 describes the extraction of copper from ammoniacal aqueous solutions by a process of extraction first with a beta-diketone followed by a second extraction with an oxime. Alternatively, there is described the use of a mixture of diketone and oxime wherein the extractant reagent comprises 5-30 percent by volume of the strong reagent (oxime) and 10-60 percent by volume of the weak reagent (beta diketone).

WO-A-93/04208 and W. Mickley And W. Uhlemann in Separation Science and Technology, 27 (12), pages 1669 - 1674 (1992) disclose a method for the recovery of copper from an aqueous ammoniacal solution containing copper values wherein the extractant employed is a beta-diketone in which at least one of the carbonyl carbon atoms is bonded to a phenyl or an alkyl substituted phenyl.

### DESCRIPTION OF THE INVENTION

It was found that when contacted with an ammoniacal solution obtained by leaching a copper sulfide concentrate with ammonia/ammonium sulfate, a preferred diketone extractant reagent, 1-phenyl-3-isoheptyl-1,3-propanedione, will extract the copper but upon repetitive use the copper may become more difficult to strip. It is believed that the problem may arise from a synergistic interaction of surfactant type materials introduced into the organic phase through a leach liquor, with a ketimine that is formed by reaction of ammonia with the beta-diketone.

It has now been discovered that the use of a sterically hindered beta-diketone, will provide a very efficient and improved process for the recovery of copper in that a significant improvement in the stability of strip kinetics will be achieved. It is believed that the steric hindrance around the beta-diketone functionality results in more stability to use conditions, minimizing, if not eliminating, ketimine formation.

By "highly sterically hindered diketone" as employed herein is meant that the diketone is highly sterically hindered where the three carbons attached to the carbonyl carbons of the diketone can together bear no more than four hydrogens when none of the three carbons is a part of a phenyl ring and no more than three hydrogens when one of the three carbons is part of a phenyl ring. It is also required that the carbon atom between the two carbonyl carbons must bear at least one hydrogen.

While the present invention is particularly useful in applications where ammoniacal leach solutions are encountered in the treatment of copper containing sulfidic ores, the present invention is applicable or useful in the extraction of copper from any aqueous ammoniacal solution containing copper values regardless of its source.

In its general application accordingly the present invention relates to a process for recovery of copper from an aqueous ammoniacal solution containing copper values comprising:
(A) contacting a copper pregnant aqueous ammoniacal solution containing copper values with a water insoluble beta-diketone copper extractant dissolved in a water-immiscible organic solvent to extract copper values from said aqueous ammoniacal solution into said organic solution thereby forming a copper pregnant organic phase and a copper depleted aqueous phase;
(B) separating said aqueous phase and said organic phase;
(C) contacting the copper pregnant organic phase with an aqueous acidic stripping solution;
(D) separating said aqueous acidic stripping solution now containing the copper values from the organic phase; and
(E) recovering the copper from said aqueous acidic stripping solution; characterized in that the beta-diketone extractant being a highly sterically hindered beta-diketone selected from beta-diketones of formula II: 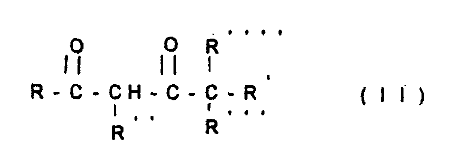
where R is phenyl or alkyl substituted phenyl, R' is alkyl having from above 1 to 8 carbon atoms, and R", R"' and R"" are the same or different and are chosen from the group consisting of H, alkyl having from 1 to 8 carbon atoms, and aralkyl having from 7 to 14 carbon atoms, with the proviso that (a) less than two of R", R"' and R"" are H and (b) the total number of carbons in all R groups is at least 13.

In a further embodiment the present invention relates to a beta-diketone extractant compound adapted for extracting copper values from aqueous ammoniacal copper containing solutions comprising a beta diketone compound of the formula II: 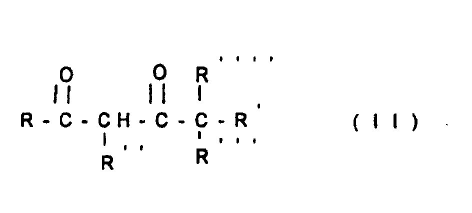 where R is phenyl or alkyl substituted phenyl, R' is alkyl having from above 1 to 8 carbon atoms, and R", R"' and R"" are the same or different and are chosen from the group consisting of H, alkyl having from 1 to 8 carbon atoms, and aralkyl having from 7 to 14 carbon atoms, with the proviso that (a) less than two of R", R"' and R"" are H and (b) the total number of carbons in all R groups is at least 13.

Referred embodiments become evident from the dependent claims.

In view thereof, the present invention also contemplates a copper extractant, which comprises a beta-diketone copper extractant having an alkyl or aralkyl substituent attached to the carbon atom which joins the two carbonyl groups of the diketone, such as 1-phenyl-2-methyl 3-isoheptyl-1,3-propanedione.

As indicated earlier, the present invention is useful in regard to aqueous ammoniacal solutions from a variety of sources such a those encountered in leaching of chalcocite concentrates. In such applications the copper pregnant leach solutions from which the copper is to be recovered by extraction will contain on the order of 15-170 g/l copper and typically 30-40 g/l copper at a pH of 8.5 to 11. In solutions encountered from other applications, the solutions may contain copper at higher levels, on the order of 125-170 g/l, such as solutions encountered in ammoniacal copper chloride printed board etchants.

Compounds of formula II are modifications of the beta-diketones found in U.S.-A-4,065,502 and 4,175,012.

Preferred compounds are those in which R is phenyl, R" is H, R' is a straight or branched chain alkyl group containing from 5-8 carbon atoms and R"' and R"" are methyl.

The various R groups in formula II are preferably free from substitution and each contains less than 20 carbon atoms.

As earlier noted when 1-phenyl-3-isoheptyl-1,3-propanedione under conditions of use is exposed to high levels of ammonia, and particularly where relatively high temperatures are encountered, such as 45 degrees Centigrade, the result is the formation of the corresponding ketimine which then results in poor stripping and may be an intermediate in a degradation pathway resulting in by-products that may contribute to very high entrainment of the aqueous phase in the loaded organic.

In the process of extraction a wide variety of water immiscible liquid hydrocarbon solvents can be used in the copper recovery process to form the organic phase in which the diketone extractant is dissolved. These include aliphatic and aromatic hydrocarbons such as kerosenes, benzene, toluene, xylene and the like. A choice of essentially water-immiscible hydrocarbon solvents or mixtures thereof will depend on factors, including the plant design of the solvent extraction plant, (mixer-settler units, extractors) and the like. The preferred solvents for use in the present invention are the aliphatic or aromatic hydrocarbons having flash points of 54°C (130 degrees Fahrenheit) and higher, preferably at least 66°C (150 degrees) and solubilities in water of less than 0.1% by weight. The solvents are essentially chemically inert. Representative commercially available solvents are Chevron™ ion exchange solvent (available from Standard Oil of California) having a flash point of 91°C (195 degrees Fahrenheit); Escaid™ 100 and 110 (available from Exxon-Europe) having a flash point of 82°C (180 degrees Fahrenheit); Norpar™ 12 (available from Exxon-USA) with a flash point of 71°C (160 degrees Fahrenheit); Conoco™ C1214 (available from Conoco) with a flash point of 71°C (160 degrees Fahrenheit); and Aromatic 150 (an aromatic kerosene available from Exxon-USA having a flash point of 66°C (150 degrees Fahrenheit)), and other various kerosenes and petroleum fractions available from other oil companies.

In the extraction process, the organic solvent solutions may contain the beta-diketone in an amount approaching 100 % solids, but typically the diketone will be employed in an amount of 20-30% by weight.

In the process, the volume ratios of organic to aqueous (O:A) phase will vary widely since the contacting of any quantity of the diketone organic solution with the copper containing aqueous ammoniacal solution will result in the extraction of copper values into the organic phase. For commercial practicality however, the organic:aqueous phase ratios for extraction are preferably in the range of 50:1 to 1:50. It is desirable to maintain an effective O:A ratio of 1:1 in the mixer unit by recycle of one of the streams. In the stripping step, the organic:aqueous stripping medium phase will preferably be in the range of 1:4 to 20:1. For practical purposes, the extracting and stripping are normally conducted at ambient temperatures and pressure although higher and lower temperatures and pressures are entirely operable. While the entire operation can be carried out as a batch operation, most advantageously the process is carried out continuously with the various streams or solutions being recycled to the various operations in the process for recovery of the copper, including the leaching, extraction and the stripping steps.

In the extraction process the extractant reagent should be soluble in the organic water-immiscible solvent. In general the diketones of the present invention will be soluble to such an extent and amount described above. If necessary or desirable to promote specific desired properties of extraction, solubility modifiers generally known in the art may be employed. Such modifiers include long chain (6-30 carbon aliphatic alcohols or esters such as n-hexanol, n-2-ethylhexanol, isodecanol, isohexadecanol, 2-(1,3,3 trimethylbutyl)-5,7,7-trimethyl octanol and 2,2,4-trimethyl-1,3-pentanediol mono-or di- isobutyrate, long chain phenols, such as heptylphenol, octylphenol, nonylphenol and dodecylphenol; and organo phosphorous compounds such as tri-lower alkyl (4-8 carbon atom) phosphates especially tributyl phosphate and tri-(2-ethylhexyl) phosphate. Where indicated to be desirable, kinetic additives may also be employed. It is preferred to avoid solubility modifiers.

The present invention also contemplates the use of a catalytic amount of an oxime, in which case the oxime functions as a kinetic additive, in combination with the sterically hindered diketones of the present invention, preferably an hydroxy, aryl oxime. By catalytic amount as employed herein, is meant a small amount of oxime in relation to the amount of diketone, preferably from .0.5 to 5 mole % of oxime relative to the beta-diketone. Such amounts of oxime are particularly effective in accelerating the rate of copper stripping from the organic phase.

The beta-diketones of this invention are also effective as co-extractants for copper from ammoniacal solutions along with strong extractants, such as oximes, a combination similar to that described in US -A- 4,350,661 noted earlier above. In such a case, the mixture of diketone and oxime in the extraction reagent will comprise 5-30 volume percent of the oxime strong reagent and 10-60 volume percent of the weaker beta-diketone.

The oxime compounds which are to be employed in catalytic amounts along with the sterically hindered beta-diketones, or may be co-extractants with the beta-diketones are certain oximes such as described in US -A- 4,563.256. Such oximes, which may be employed are those generally conforming to the formula: 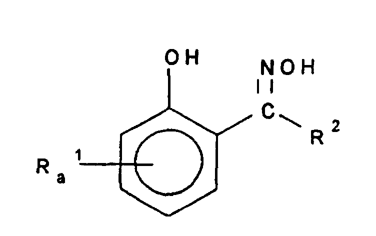 where R¹ is a saturated aliphatic group of 1-25 carbon atoms or an ethylenically unsaturated group of 3-25 carbon atoms or OR³, where R³ is a saturated or ethylenically unsaturated group as defined above, a is an integer of 0, 1, 2, 3 or 4 and R² is H, or a saturated or ethylenically unsaturated group as defined above, with the proviso that the total number of carbon atoms in R¹ and R² is from 3-25, or is phenyl or R⁴ substituted phenyl where R⁴ is a saturated or ethylenically unsaturated group as defined above, which may be the same or different from R¹. Illustrative of some of the oxime compounds are 5-heptyl salicylaldoxime, 5-octyl salicylaldoxime, 5-nonyl salicylaldoxime, 5-dodecyl salicylaldoxime, 5-nonyl-2-hydroxyacetophenone oxime, 5-dodecyl-2-hydroxyacetophenone oxime, 2-hydroxy-5-nonyl benzophenone oxime and 2-hydroxy-5-dodecyl benzophenone oxime. While it may be preferred that a single oxime compound be employed along with the beta-diketone, mixtures of oximes may be employed to meet particular system requirements.

In the stripping step, a sulfuric acid solution containing 60-180 g/l sulfuric acid is the preferred stripping agent as it permits the subsequent recovery of the copper by conventional recovery steps either in the form of copper sulphate crystals or by electrowinning to cathode copper. Other mineral acids may be used such as hydrochloric or nitric; however, such may require other recovery methods or specialized handling equipment.

Hindered beta-diketones may be prepared by the method of Example A of U.S. -A- 4,065,502. Thus, methyl 2,2-dimethyl octanoate is condensed with acetophenone to give 1-phenyl-4,4-dimethyl-1,3-undecanedione.

These hindered beta-diketones are then employed in a process for the recovery and extraction of copper from an aqueous ammoniacal copper containing solution as described earlier above in steps (A) through (E).

## Claims

1. A process for recovery of copper from an aqueous ammoniacal solution containing copper values comprising:
(A) contacting a copper pregnant aqueous ammoniacal solution containing copper values with a water insoluble beta-diketone copper extractant dissolved in a water-immiscible organic solvent to extract copper values from said aqueous ammoniacal solution into said organic solution thereby forming a copper pregnant organic phase and a copper depleted aqueous phase;
(B) separating said aqueous phase and said organic phase;
(C) contacting the copper pregnant organic phase with an aqueous acidic stripping solution;
(D) separating said aqueous acidic stripping solution now containing the copper values from the organic phase; and
(E) recovering the copper from said aqueous acidic stripping solution; **characterized in that** the beta-diketone extractant being a highly sterically hindered beta-diketone selected from beta-diketones of formula II: 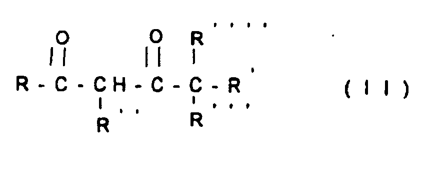
where R is phenyl or alkyl substituted phenyl, R' is alkyl having from above 1 to 8 carbon atoms, and R", R"' and R"" are the same or different and are chosen from the group consisting of H, alkyl having from 1 to 8 carbon atoms, and aralkyl having from 7 to 14 carbon atoms, with the proviso that (a) less than two of R", R"' and R"" are H and (b) the total number of carbons in all R groups is at least 13.

2. A process as defined in claim 1, wherein R is phenyl, R" is H, R' is a branched chain alkyl group having from 5-8 carbon atoms and R"' and R"" are methyl.

3. A process as defined in claim 1 wherein R" is selected from the group consisting of methyl and benzyl.

4. A process as defined in claim 1 wherein the diketone extractant is 1-phenyl-2-methyl-3-isoheptyl-1,3-propanedione.

5. A process as defined in claim 1 wherein the diketone extractant is 1-phenyl-2-benzyl-3-isoheptyl-1,3-propanedione.

6. A process as defined in claim 1 wherein the diketone is 1-phenyl-4,4-dimethyl-1,3-undecanedione.

7. A process as defined in claim 1, in which the extractant further comprises an hydroxy aryl oxime.

8. A process as defined in claim 7, wherein said hydroxy aryl oxime is present in a catalytic amount.

9. A process as defined in claim 7, wherein said hydroxyaryl oxime is a co-extractant with the beta-diketone.

10. A process as defined in claim 7, wherein said hydroxy aryl oxime has the formula: 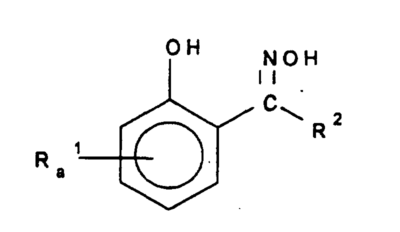 where R' is a saturated aliphatic group of 1-25 carbon atoms or an ethylenically unsaturated group of 3-25 carbon atoms or OR³, where R³ is a saturated or ethylenically unsaturated group as defined above, a is an integer of 0, 1, 2, 3 or 4 and R² is H, or a saturated or ethylenically unsaturated group as defined above, with the proviso that the total number of carbon atoms in R¹ and R² is from 3-25, or is phenyl or R⁴ substituted phenyl where R⁴ is a saturated or ethylenically unsaturated group as defined above, which may be the saure or different from R¹.

11. A process as defined in claim 10, in which said hydroxy aryl oxime is selected from the group consisting of 5-heptyl salicylaldoxime, 5-octyl salicylaldoxime, 5-nonyl salicylaldoxime, 5-dodecyl salicylaldoxime, 5-nonyl-2-hydroxyacetophenone oxime, 5-dodecyl-2-hydroxyacetophenone oxime, 2-hydroxy-5-nonyl benzophenone oxime and 2-hydroxy-5-dodecyl benzophenone oxime.

12. A beta-diketone extractant compound adapted for extracting copper values from aqueous ammoniacal copper containing solutions comprising a beta diketone compound of the formula II: 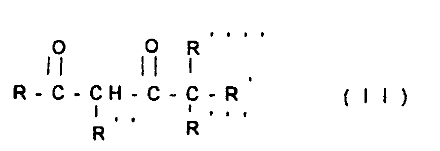 where R is phenyl or alkyl substituted phenyl, R' is alkyl having from above 1 to 8 carbon atoms, and R", R"' and R"" are the same or different and are chosen from the group consisting of H, alkyl having from 1 to 8 carbon atoms, and aralkyl having from 7 to 14 carbon atoms, with the proviso that (a) less than two of R", R"' and R"" are H and (b) the total number of carbons in all R groups is at least 13.

13. A beta-diketone compound as defined in claim 12, in which R" is selected from the group consisting of methyl and benzyl.

14. A beta-diketone compound as defined in claim 12, wherein R is phenyl, R" is methyl or benzyl, R' is branched hexyl and R"' and R"" are H.

15. A beta-diketone compound as defined in claim 12, wherein R is phenyl, R" is H, R' is a branched chain alkyl group having from 5-8 carbon atoms and R"' and R"" are methyl.

16. A beta diketone according to claim 12 selected from 1-phenyl-2-benzyl-3-isoheptyl-1,3-propanedione, 1-phenyl-2-methyl-3-isoheptyl-1,3-propanedione and 1-phenyl-4,4-dimethyl-1,3-undecanedione.

## Patentansprüche

1. Verfahren zur Gewinnung von Kupfer aus einer wässrigen ammoniakalischen Lösung, die wertvolle Kupferanteile enthält, umfassend:
(A) das In-Kontakt-Bringen einer kupferreichen, wässrigen, ammoniakalischen Lösung, die wertvolle Kupferanteile enthält, mit einem in Wasser unlöslichen β-Diketon-Kupferextraktionsmittel, das in einem mit Wasser nicht mischbaren organischen Lösungsmittel gelöst ist, um wertvolle Kupferanteile aus dieser wässrigen ammoniakalischen Lösung in die organische Lösung zu extrahieren, wodurch eine kupferreiche organische Phase und eine an Kupfer verarmte wässrige Phase gebildet werden;
(B) das Trennen der wässrigen Phase und der organischen Phase;
(C) das In-Kontakt-Bringen der kupferreichen organischen Phase mit einer wässrigen sauren Stripping-Lösung;
(D) das Trennen der wässrigen sauren Stripping-Lösung, die jetzt die wertvollen Kupferanteile enthält, von der organischen Phase; und
(E) das Gewinnen des Kupfers aus der wässrigen sauren Stripping-Lösung;
**dadurch gekennzeichnet, dass** das β-Diketon-Extraktionsmittel ein stark sterisch gehindertes β-Diketon ist, das aus β-Diketonen der Formel II: 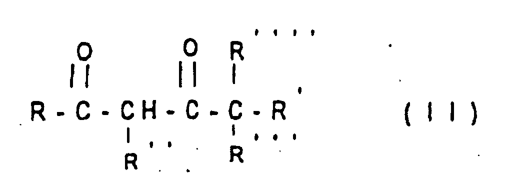 ausgewählt ist, wobei R Phenyl oder alkylsubstituiertes Phenyl ist, R' Alkyl mit mehr als 1 bis 8 Kohlenstoffatomen ist, und R", R"' und R"" einander gleich oder voneinander verschieden sind und aus der Gruppe ausgewählt sind, die aus H, Alkyl mit 1 bis 8 Kohlenstoffatomen und Aralkyl mit 7 bis 14 Kohlenstoffatomen besteht, mit der Maßgabe, dass (a) weniger als zwei von R", R"' und R"" H sind und (b) die Gesamtzahl der Kohlenstoffatome in allen R-Gruppen wenigstens 13 ist.

2. Verfahren gemäß Anspruch 1, wobei R Phenyl ist, R" H ist, R' eine verzweigtkettige Alkylgruppe mit 5 bis 8 Kohlenstoffatomen ist, und R"' und R"" Methyl sind.

3. Verfahren gemäß Anspruch 1, wobei R" aus der aus Methyl und Benzyl bestehenden Gruppe ausgewählt ist.

4. Verfahren gemäß Anspruch 1, wobei das Diketon-Extraktionsmittel 1-Phenyl-2-methyl-3-isoheptyl-1,3-propandion ist.

5. Verfahren gemäß Anspruch 1, wobei das Diketon-Extraktionsmittel 1-Phenyl-2-benzyl-3-isoheptyl-1,3-propandion ist.

6. Verfahren gemäß Anspruch 1, wobei das Diketon 1-Phenyl-4,4-dimethyl-1,3-undecandion ist.

7. Verfahren gemäß Anspruch 1, wobei das Extraktionsmittel weiterhin ein Hydroxyaryloxim umfasst.

8. Verfahren gemäß Anspruch 7, wobei das Hydroxyaryloxim in einer katalytischen Menge vorliegt.

9. Verfahren gemäß Anspruch 7, wobei das Hydroxyaryloxim ein Co-Extraktionsmittel mit dem β-Diketon ist.

10. Verfahren gemäß Anspruch 7, wobei das Hydroxyaryloxim die Formel: 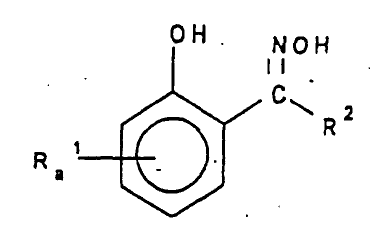 aufweist, wobei R¹ eine gesättigte aliphatische Gruppe mit 1 bis 25 Kohlenstoffatomen oder eine ethylenisch ungesättigte Gruppe mit 3 bis 25 Kohlenstoffatomen oder OR³ ist, wobei R³ eine wie oben definierte gesättigte oder ethylenisch ungesättigte Gruppe ist, a eine ganze Zahl von 0, 1, 2, 3 oder 4 ist, und R² H oder eine wie oben definierte gesättigte oder ethylenisch ungesättigte Gruppe ist, mit der Maßgabe, dass die Gesamtzahl der Kohlenstoffatome in R¹ und R² 3 bis 25 ist, oder R² Phenyl oder R⁴-substituiertes Phenyl ist, wobei R⁴ eine wie oben definierte gesättigte oder ethylenisch ungesättigte Gruppe ist, die mit R¹ identisch sein kann oder von R¹ verschieden sein kann.

11. Verfahren gemäß Anspruch 10, wobei das Hydroxyaryloxim aus der Gruppe ausgewählt ist, bestehend aus 5-Heptylsalicylaldoxim, 5-Octylsalicylaldoxim, 5-Nonylsalicylaldoxim, 5-Dodecylsalicylaldoxim, 5-Nonyl-2-hydroxyacetophenonoxim, 5-Dodecyl-2-hydroxyacetophenonoxim, 2-Hydroxy-5-nonylbenzophenonoxim und 2-Hydroxy-5-dodecylbenzophenonoxim.

12. β-Diketon-Extraktionsmittel, das zur Extraktion von wertvollen Kupferanteilen aus wässrigen ammoniakalischen, kupferhaltigen Lösungen, umfassend eine β-Diketon-Verbindung der Formel II: 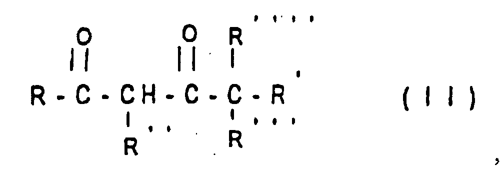 befähigt ist, wobei R Phenyl oder alkylsubstituiertes Phenyl ist, R' Alkyl mit mehr als 1 bis 8 Kohlenstoffatomen ist, und R", R"' und R"" einander gleich oder voneinander verschieden sind und aus der Gruppe ausgewählt sind, die aus H, Alkyl mit 1 bis 8 Kohlenstoffatomen und Aralkyl mit 7 bis 14 Kohlenstoffatomen besteht, mit der Maßgabe, dass (a) weniger als zwei von R", R"' und R"" H sind und (b) die Gesamtzahl der Kohlenstoffatome in allen R-Gruppen wenigstens 13 ist.

13. β-Diketon-Verbindung gemäß Anspruch 12, wobei R" aus der aus Methyl und Benzyl bestehenden Gruppe ausgewählt ist.

14. β-Diketon-Verbindung gemäß Anspruch 12, wobei R Phenyl ist, R" Methyl oder Benzyl ist, R' verzweigtes Hexyl ist, und R"' und R"" H sind.

15. β-Diketon-Verbindung gemäß Anspruch 12, wobei R Phenyl ist, R" H ist, R' eine verzweigtkettige Alkylgruppe mit 5 bis 8 Kohlenstoffatomen ist, und R"' und R"" Methyl sind.

16. β-Diketon gemäß Anspruch 12, das aus 1-Phenyl-2-benzyl-3-isoheptyl-1,3-propandion, 1-Phenyl-2-methyl-3-isoheptyl-1,3-propandion und 1-Phenyl-4,4-dimethyl-1,3-undecandion ausgewählt ist.

## Revendications

1. Procédé de récupération du cuivre à partir d'une solution aqueuse ammoniacale contenant des teneurs ("values") de cuivre, comprenant :
(A) la mise en contact d'une solution aqueuse ammoniacale chargée en cuivre contenant des teneurs de cuivre avec un extractant du cuivre de type bêta-dicétone insoluble dans l'eau dissous dans un solvant organique non miscible dans l'eau pour extraire des teneurs de cuivre de ladite solution aqueuse ammoniacale dans ladite solution organique et ainsi la formation d'une phase organique chargée en cuivre et d'une phase aqueuse appauvrie en cuivre ;
(B) la séparation de ladite phase aqueuse et de ladite phase organique ;
(C) la mise en contact de la phase organique chargée en cuivre avec une solution aqueuse acide d'extraction ;
(D) la séparation de ladite solution aqueuse acide d'extraction contenant maintenant les teneurs de cuivre de la phase organique ;
(E) la récupération du cuivre de ladite solution aqueuse acide d'extraction ; **caractérisé en ce que** l'extractant de type bêta-dicétone est une bêta-dicétone à fort encombrement stérique choisie parmi les bêtadicétones de formule II : 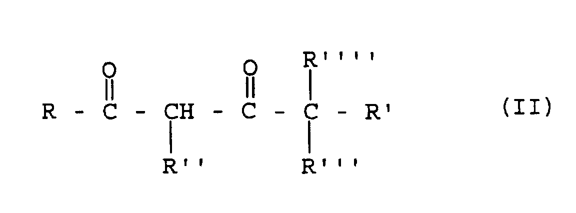
dans laquelle R est un phényle ou un phényle substitué par un alkyle, R' est un alkyle ayant de 1 à 8 atomes de carbone, et R'', R''' et R'''' sont identiques ou différents et sont choisis dans le groupe constitué par H, un alkyle ayant de 1 à 8 atomes de carbone, et un aralkyle ayant de 7 à 14 atomes de carbone, à la condition que (a) moins de deux éléments parmi R'', R"' et R'''' soient H et (b) le nombre total de carbones dans tous les groupes R soit au moins égal à 13.

2. Procédé selon la revendication 1, dans lequel R est un phényle, R" est H, R' est un groupe alkyle à chaîne ramifiée ayant de 5 à 8 atomes de carbone et R''' et R'''' sont un méthyle.

3. Procédé selon la revendication 1, dans lequel R'' est choisi dans le groupe constitué par le méthyle et le benzyle.

4. Procédé selon la revendication 1, dans lequel l'extradant de type dicétone est la 1-phényl-2-méthyl-3-isoheptyl-1,3-propanedione.

5. Procédé selon la revendication 1, dans lequel l'extractant de type dicétone est la 1-phényl-2-benzyl-3-isoheptyl-1,3-propanedione.

6. Procédé selon la revendication 1, dans lequel la dicétone est la 1-phényl-4,4-diméthyl-1,3-undécanedione.

7. Procédé selon la revendication 1, dans lequel l'extractant comprend en outre une hydroxyaryloxime.

8. Procédé selon la revendication 7, dans lequel ladite hydroxyaryloxime est présente dans une quantité catalytique.

9. Procédé selon la revendication 7, dans lequel ladite hydroxyaryloxime est un co-extractant avec la bêta-dicétone.

10. Procédé selon la revendication 7, dans lequel ladite hydroxyaryloxime a la formule : 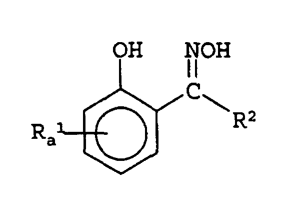 dans laquelle R' est un groupe aliphatique saturé ayant de 1 à 25 atomes de carbone ou un groupe éthyléniquement insaturé ayant de 3 à 25 atomes de carbone ou OR³, R³ étant un groupe saturé ou éthyléniquement insaturé tel que défini ci-dessus, a est un entier égal à 0, 1, 2, 3 ou 4 et R² est H, ou un groupe saturé ou éthyléniquement insaturé tel que défini ci-dessus, à la condition que le nombre total d'atomes de carbone dans R¹ et R² soit de 3 à 25, ou est un phényle ou un phényle substitué par R⁴, R⁴ étant un groupe saturé ou éthyléniquement insaturé tel que défini ci-dessus, lequel peut être identique ou différent de R¹.

11. Procédé selon la revendication 10, dans lequel ladite hydroxyaryloxime est choisie dans le groupe constitué par la 5-heptyl-salicylaldoxime, la 5-octyl-salicylaldoxime, la 5-nonyl-salicylaldoxime, la 5-dodécyl-salicylaldoxime, la 5-nonyl-2-hydroxyacétophénone-oxime, la 5-dodécyl-2-hydroxyacétophénone-oxime, la 2-hydroxy-5-nonylbenzophénone-oxime et la 2-hydroxy-5-dodécylbenzophénone-oxime.

12. Composé extractant de type bêta-dicétone adapté à l'extraction de teneurs ("values") de cuivre de solutions aqueuses ammoniacales contenant du cuivre comprenant un composé de bêta-dicétone de formule II : 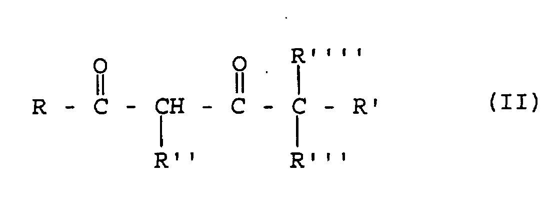 dans laquelle R est un phényle ou un phényle substitué par un alkyle, R' est un alkyle ayant de plus de 1 à 8 atomes de carbone, et R", R''' et R'''' sont identiques ou différents et sont choisis dans le groupe constitué par H, un alkyle ayant de 1 à 8 atomes de carbone, et un aralkyle ayant de 7 à 14 atomes de carbone, à la condition que (a) moins de deux éléments parmi R", R''' et R'''' soient H et (b) le nombre total de carbones dans tous les groupes R soit au moins égal à 13.

13. Composé de bêta-dicétone selon la revendication 12, dans lequel R" est choisi dans le groupe constitué par le méthyle et le benzyle.

14. Composé de bêta-dicétone selon la revendication 12, dans lequel R est un phényle, R" est un méthyle ou un benzyle, R' est un hexyle ramifié et R''' et R'''' sont H.

15. Composé de bêta-dicétone selon la revendication 12, dans lequel R est un phényle, R" est H, R' est un groupe alkyle à chaîne ramifiée ayant de 5 à 8 atomes de carbone et R''' et R'''' sont un méthyle.

16. Bêta-dicétone selon la revendication 12, choisie parmi la 1-phényl-2-benzyl-3-isoheptyl-1,3-propanedione, la 1-phényl-2-méthyl-3-isoheptyl-1,3-propanedione et la 1-phényl-4,4-diméthyl-1,3-undécanedione.
